# EUROPÄISCHE PATENTSCHRIFT

(11) **EP 1 397 384 B1**
(45) Veröffentlichungstag und Bekanntmachung des Hinweises auf die Patenterteilung: **19.04.2006**
(21) Anmeldenummer: 02751006.4
(22) Anmeldetag: 07.06.2002
(51) Int. Cl.: C07K 14/47, A61P 31/00

(54) **ANTIMIKROBIELL WIRKENDES PEPTID**
ANTIMICROBIALLY ACTIVE PEPTIDE
PEPTIDE A ACTION ANTIMICROBIENNE

(30) Priorität: 13.06.2001 DE 10129983
(43) Veröffentlichungstag der Anmeldung: 17.03.2004
(73) Patentinhaber: Eberhard-Karls-Universität Tübingen Universitätsklinikum, 72076 Tübingen (DE)
(72) Erfinder: GARBE, Claus, 72074 Tübingen (DE); SCHITTEK, Birgit, 70563 Stuttgart (DE)
(74) Vertreter: Otten, Hajo
(86) Internationale Anmeldenummer: PCT/EP2002/006238
(87) Internationale Veröffentlichungsnummer: WO 2002/100895

(56) Entgegenhaltungen:
- WO-A-99/64439
- US-A- 5 834 192
- SCHROEDER J.M.: "epithelial peptide antibiotics" BIOCHEMICAL PHARMACOLOGY, Bd. 57, 1999, Seiten 121-134, XP002248275
- SCHITTEK B ET AL: "DERMICIDIN: A NOVEL HUMAN ANTIBIOTIC PEPTIDE SECRETED BY SWEAT GLANDS" NATURE IMMUNOLOGY, NATURE PUBLISHING GROUP,, GB, Bd. 2, Nr. 12, Dezember 2001 (2001-12), Seiten 1133-1137, XP001094755 ISSN: 1529-2908

### SEQUENZPROTOKOLL

<110> Eberhard-Karls-Universität Tü Universitätsklinikum
<120> Antimikrobiell wirkendes Peptid
<130> 5402P204
<140>
   <141>
<160> 3
<170> PatentIn Ver. 2.1
<210> 1
   <211> 110
   <212> PRT
   <213> Humanes DCD-Protein aus Schweiß
<400> 1
   Met Arg Phe Met Thr Leu Leu Phe Leu Thr Ala Leu Ala Gly Ala Leu
     1               5                  10                  15
   Val Cys Ala Tyr Asp Pro Glu Ala Ala Ser Ala Pro Gly Ser Gly Asn
                20                  25                  30
   Pro Cys His Glu Ala Ser Ala Ala Gln Lys Glu Asn Ala Gly Glu Asp
            35                  40                  45
   Pro Gly Leu Ala Arg Gln Ala Pro Lys Pro Arg Lys Gln Arg Ser Ser
        50                  55                  60
   Leu Leu Glu Lys Gly Leu Asp Gly Ala Lys Lys Ala Val Gly Gly Leu
    65                  70                  75                  80
   Gly Lys Leu Gly Lys Asp Ala Val Glu Asp Leu Glu Ser Val Gly Lys
                    85                  90                  95
   Gly Ala Val His Asp Val Lys Asp Val Leu Asp Ser Val Leu
               100                 105                 110
<210> 2
   <211> 48
   <212> PRT
   <213> Künstliche Sequenz
<220>
   <223> Beschreibung der künstlichen Sequenz: Antimikrobiell wirkendes Fragment (Pos. 63-110) vom DCD-Protein SEQ ID Nr 1
<400> 2
   Ser Ser Leu Leu Glu Lys Gly Leu Asp Gly Ala Lys Lys Ala Val Gly
   1               5                   10                  15
   Gly Leu Gly Lys Leu Gly Lys Asp Ala Val Glu Asp Leu Glu Ser Val
               20                  25                  30
   Gly Lys Gly Ala Val His Asp Val Lys Asp Val Leu Asp Ser Val Leu
           35                  40                  45
<210> 3
   <211> 47
   <212> PRT
   <213> Künstliche Sequenz
<220>
   <223> Beschreibung der künstlichen Sequenz: Antimikrobiell wirkendes Fragment (Pos. 63-109) vom DCD-Protein SEQ ID Nr 1
<400> 3
   Ser Ser Leu Leu Glu Lys Gly Leu Asp Gly Ala Lys Lys Ala Val Gly
   1               5                   10                  15
   Gly Leu Gly Lys Leu Gly Lys Asp Ala Val Glu Asp Leu Glu Ser Val
               20                  25                  30
   Gly Lys Gly Ala Val His Asp Val Lys Asp Val Leu Asp Ser Val
           35                  40                  45

## Beschreibung

Die vorliegende Erfindung betrifft ein antimikrobiell wirkendes Peptid sowie dessen Verwendung und Herstellung.

Das Epithelgewebe von Säugetieren stellt eine wichtige Barriere gegenüber der Umgebung dar und liefert eine erste Verteidigungslinie gegenüber eindringenden Mikroorganismen. Insbesondere antimikrobielle Peptide, die sich zahlreich in der Epidermis finden, nehmen an dem Verteidigungssystem teil. Sie kontrollieren mikrobielles Wachstum in den ersten Stunden nach einer epithelialen Verletzung und während der Wundheilung. Insbesondere sind sie bei einigen entzündlichen Hautkrankheiten zu finden.

In der Säugerhaut sind bisher zwei Klassen von antimikrobiellen Peptiden entdeckt worden, die Cathelicidine und die β-Defensine. Sie werden nach Induktion durch entzündliche Stimuli in humanen Kreatinozyten induziert und wirken in erster Linie als Reaktion auf Verletzungen und nicht im Rahmen einer konstanten Modulation des epithelialen Verteidigungsmechanis-mus.

Während beispielsweise das Cathelicidin PR-39 eine Komponente in der Wundflüssigkeit ist und an der Wundheilung beteiligt zu sein scheint, wird das Cathelicidin LL-37 in humanen Hautkreatinozyten an entzündlichen Stellen bei verschiedenen Krankheiten exprimiert.

Defensine sind kleine kationische Peptide mit einem Molekulargewicht von 3 bis 5 kDa, sie haben eine antibakterielle sowie eine antimykotische Wirkung. Die α-Defensine HD1-4 werden beispielsweise in humanen Neutrophilen exprimiert, die sich in infizierten Gewebebereichen ansammeln. Die α-Defensine HD-5 und HD-6 werden dagegen von epithelialen Granulozyten produziert.

Allgemein sind antimikrobielle Peptide endogene, Gen-kodierte Peptide mit besonderer Bedeutung für die frühe Phase der Abwehr gegen mikrobielle Erreger. Sie können innerhalb von Minuten bis Stunden nach dem ersten Kontakt mit dem Pathogen nachgewiesen werden.

Allerdings wirken die bekannten antimikrobiellen Peptide nicht gegen alle mikrobiellen Pathogene in gleicher Weise, Defensine wirken beispielsweise nur ungenügend bei Infektionen mit S. aureus, einer wichtigen Ursache für Hautinfektionen, insbesondere bei atopischer Dermatitis.

Für die Bekämpfung pathogener Mikroorganismen werden präventiv oder kurativ auch Antibiotika eingesetzt, also Stoffe mikrobiologischer Herkunft, die andere Mikroorganismen an ihrem Wachstum hemmen oder sogar abtöten. Im Gegensatz zu den oben erwähnten Cathelicidinen und Defensinen haben Antibiotika in der Regel eine selektive Wirksamkeit. Viele Mikroorganismen besitzen eine natürliche Unempfindlichkeit gegenüber einem Antibiotikum, sie können diese sogenannte Antibiotika-Resistenz aber auch im Verlauf des Wachstums in Gegenwart von Antibiotika entwickeln.

Durch Mutations- und Selektionsprozesse sowie durch die Ausbildung von Resistenzen ergeben sich nicht nur im Klinikalltag sondern auch bei der Arzneimittel- und Kosmetikaherstellung immer häufiger Probleme mit mikrobiellen Pathogenen, die nicht oder nicht effektiv bekämpft werden können.

Vor diesem Hintergrund besteht ein ständiger Bedarf an neuen antimikrobiell wirkenden Agenzien, die präventiv oder kurativ eingesetzt werden können.

Vor diesem Hintergrund liegt der vorliegenden Erfindung die Aufgabe zugrunde, ein weiteres antimikrobiell wirkendes Peptid bereitzustellen und einen Weg zu seiner Produktion aufzuzeigen.

Erfindungsgemäß wird diese Aufgabe gelöst durch ein antimikrobiell wirkendes Peptid, das ein Fragment von höchstens 50 Aminosäureresten aus dem C-terminalen Bereich des DCD-Proteins mit der Sequenz SEQ ID Nr: 1 aus dem beiliegenden Sequenzprotokoll umfaßt.

Die der Erfindung zugrunde liegende Aufgabe wird auf diese Weise vollkommen gelöst.

Die Erfinder der vorliegenden Anmeldung konnten nämlich in einer cDNA-Bibliothek für Haut ein Gen identifizieren, das sie Dermcidin (im folgenden: DCD) genannt haben. Das Gen besteht aus fünf Exons und vier Introns und ist auf dem Chromosom 12q13 zwischen den Markern D12S1896 und D12S1632 (lod score 14.11) lokalisiert.

DCD hat ein sehr restriktives Expressionsmuster, da das Gen nur in humaner Haut exprimiert wird und weder in 50 analysierten humanen Geweben verschiedenen Ursprungs noch in humanen Fibroblasten, Keratinozyten, Melanozyten oder Melanomzellinien detektiert werden konnte.

Das Gen kodiert ein Peptid, das zu einer neuen Klasse von antimikrobiellen Proteinen gehört und eine Breitbandaktivität aufweist. Das Peptid wird spezifisch in Schweißdrüsen exprimiert, in den Schweiß sekretiert und auf die epidermale Oberfläche transportiert. Im Schweiß wird es proteolytisch zu einem Peptid prozessiert, das eine dosisabhängige, antimikrobielle Wirkung gegen eine Vielzahl von pathogenen Mikroorganismen aufweist. Mit einem antimikrobiellen Assay konnten die Erfinder zeigen, das das DCD-Protein toxisch für Escherichia coli, Enterococcus faecalis, Staphylococcus aureus und Candida albicans ist.

Bis zur vorliegenden Erfindung waren im humanen Schweiß keine antimikrobiellen Peptide entdeckt worden. Aus der Entdeckung des DCD sowie dem experimentellen Nachweis, daß DCD sowie Fragmente des DCD antimikrobiell wirken, folgern die Erfinder der vorliegenden Anmeldung, daß Schweiß eine Rolle in der Regulation der humanen Hautflora spielt und daß DCD eine therapeutische Bedeutung für die Behandlung von Hautkrankheiten hat. Im Schweiß kommt DCD in einer Menge von 1-10 µg/ml vor, wobei genau dieser Konzentrationsbereich im experimentellen Ansatz eine antimikrobielle Wirkung gegen die obengenannten Pathogene entfaltet.

Ein Vergleich mit der Genbank ergab überraschenderweise, daß die cDNA-Sequenz von DCD von Akerblom et al. als "human cachexia associated protein" (HCAP) veröffentlicht wurde. Die Autoren beschreiben in US 5,834,192 die Identifizierung und Isolierung von HCAP aus einer Brusttumorbibliothek und schlagen die therapeutsche Anwendung von HCAP sowie des kodierenden Gens im Rahmen einer Behandlung von Tumor-induzierter Cachexie vor. Eine antimikrobielle Wirkung von HCAP wird nicht offenbart.

Interessanterweise weist ein kurzer Abschnitt im N-terminalen Bereich von DCD - Aminosäurenreste 20-49 - eine 96prozentige Homologie zu einem Y-P30 genannten "survival promoting peptide" auf; siehe Cunningham et al.: "Calreticulin Binding and Other Biological Activities of Survival Peptide Y-P30 Including Effects of Systemic Treatment of Rats", Experimental Neurology 163, 254-268 (2000).

Y-P30 wurde von oxidativ beanspruchten neuralen Zellinien aufgereinigt und hat offenbar einen das Überleben begünstigenden Effekt auf Neuronen, da eine direkte Applikation dieses Peptides zu Läsionen des zerebralen Cortex in Ratten Neuronen überleben ließ, die nach einer Cortexläsion üblicherweise degenerieren. Die Autoren schlagen vor, daß Y-P30 von neuralen Zellen zum Zwecke der Zytoprotektion als Antwort auf Streß sekretiert wird.

Das erfindungsgemäße Peptid umfaßt ein Fragment von höchstens 50 Aminosäureresten aus dem C-terminalen Bereich von DCD, vorzugsweise entweder die Aminosäurereste 63-110 (SEQ ID Nr: 2) oder die Aminosäurereste 63-109 (SEQ ID Nr: 3).

Es hat sich gezeigt, daß diese Fragmente eine hervorragende antimikrobielle Wirkung haben, insbesondere gegenüber den oben erwähnten Pathogenen. Da diese Fragmente auch deutlich kürzer sind als das reife DCD-Protein, das 110 Aminosäurereste umfaßt, wobei die ersten 19 N-terminalen Aminosäurereste ein Signalpeptid darstellen, lassen sie sich sowohl durch chemische Synthese als auch biotechnologisch leichter und preiswerter herstellen als das reife DCD-Protein. Die geringere Größe der Fragmente verglichen mit dem reifen DCD-Protein hat neben der leichteren und preiswerteren Herstellbarkeit aber auch den weiteren Vorteil, daß kürzere Fragmente in der Regel stabiler sind als längere, so daß auch die Handhabung sowie die Applikation bei den Fragmenten gegenüber dem reifen Protein einfacher ist und damit weitere Vorteile aufweist.

Auch eine weitere Verkürzung der beiden Fragmente SEQ ID Nr: 2 und SEQ ID Nr: 3 am N-terminalen Ende beeinträchtigt die antimikrobielle Wirkung nicht, während die Erfinder feststellen konnten, daß eine Verkürzung auf die 31 C-terminalen Aminosäurereste von DCD zu einem Peptid führt, das keine nennenswerte antimikrobielle Wirkung mehr aufweist.

Weiter ist es bevorzugt, wenn verglichen mit der entsprechenden Position im reifen DCD-Protein zumindest eine Aminosäure gegen eine Aminosäure der gleichen Gruppe ausgetauscht ist.

Es ist bekannt, daß sich die sogenannten proteinogenen Aminosäuren in vier Gruppen einteilen lassen, und daß der Austausch einer Aminosäure in einem Peptid gegen eine Aminosäure der gleichen Gruppe die Funktion des Peptides häufig nicht oder nur geringfügig verändert. Ein solcher Aminosäureaustausch kann insbesondere im Hinblick auf eine chemische Synthese oder eine biotechnologische Herstellung sinnvoll sein, wenn sich aufgrund des Austausches das entsprechende Peptid mit einer höheren Ausbeute herstellen läßt, wobei aufgrund des Austausches innerhalb einer Gruppe die antimikrobielle Wirkung erhalten bleibt.

Lediglich der Vollständigkeit halber sei erwähnt, daß die Aminosäuregruppen wie folgt charakterisiert sind: I. Aminosäuren mit neutralen und hydrophoben (unpolaren) Seitenketten, II. Aminosäuren mit neutralen und hydrophylen (polaren) Seitenketten, III. Aminosäuren mit sauren und hydrophylen (polaren) Seitenketten und IV. Aminosäuren mit basischen und hydrophylen (polaren) Seitenketten.

Die Erfindung betrifft ferner ein Peptid, das eine zu dem neuen Peptid homologe Aminosäuresequenz aufweist und eine vergleichbare antimikrobielle Wirkung zeigt.

Unter einem homologen Peptid wird im Rahmen der vorliegenden Erfindung ein Peptid verstanden, das durch divergente Evolution aus einem gemeinsamen Vorläufer von DCD entstanden ist und eine große Übereinstimmung nicht nur in der Primär- sondern auch in der Sekundär- und Tertiärstruktur aufweist, biologisch vergleichbar gebildet wird und eine vergleichbare Funktion hat. Nachdem die Erfinder nämlich zeigen konnten, daß das in humanen Schweißdrüsen exprimierte, sekretierte und prozessierte DCD-Protein bzw. C-terminale Fragmente des DCD-Proteins, die teilweise in humanem Schweiß natürlich vorkommen, eine antimikrobielle Wirkung aufweisen, können entsprechende homologe Peptide bei anderen Säugetieren problemlos aufgefunden werden. Ausgehend von der überraschenden Erkenntnis, daß es im humanen Schweiß zumindest ein antibakteriell wirkendes Peptid gibt, sind die Schritte für das Auffinden homologer Peptide auch in anderen Säugetieren so weit vorgezeichnet, daß sie von der vorliegenden Erfindung mit umfaßt sind.

In einer Weiterbildung ist es bevorzugt, wenn das Peptid zumindest eine posttranslationelle Modifizierung aufweist.

Unter posttranslationeller Modifizierung wird im Rahmen der vorliegenden Anmeldung insbesondere die Anheftung von prosthetischen Gruppen (beispielsweise Glycosylierung) und die Modifizierung von Aminosäureresten (beispielsweise Alkylierung) verstanden. Im allgemeinen Sinne ist also mit einer Posttranslationsmodifizierung jeder Unterschied zwischen dem erfindungsgemäß eingesetzten, funktionellen Peptid und der linearen Abfolge der unveränderten Aminosäurereste gemeint.

Derartige posttranslationelle Modifizierungen können der Stabilität des Peptides oder einer gesteigerten biologischen Wirksamkeit dienen, aber auch auf eine biotechnologische Herstellung zurückzuführen sein. So führt eine Produktion von Peptiden in prokariontischen Zellen zu einer reduzierten Form des Peptides, während eine Produktion in eukäriontischen Zellen zu einem glycosylierten Peptid führen kann. Darüber hinaus kann es sinnvoll sein, zumindest eine der Aminosäuren des Peptides mit einer Schutzgruppe zu versehen, um das Peptid vor dem Angriff von Exopeptidasen zu schützen.

In einem Ausführungsbeispiel ist es bevorzugt, wenn das Peptid mit einem weiteren Peptid oder Protein zu einem Fusionsprotein verbunden ist, wobei das weitere Peptid oder Protein vorzugsweise ausgewählt ist aus der Gruppe: Signalpeptid, Reporterprotein, Histidin-Tags, antigene Determinanten etc.

Wenn Peptide als Fusionspeptide synthetisiert werden, kann die Herstellung und Reinigung der erfindungsgemäßen Peptide erleichtert werden. Für Aminosäureabschnitte oder Domänen bekannter Proteine kodierende Sequenzen werden dabei an für die erfindungsgemäßen Peptide kodierenden Nukleinsäuren anfusioniert, so daß bei der Expression ein durchgehendes Peptid erzeugt wird. Beispiele für solche anfusionierten Aminosäureabschnitte sind beispielsweise die Histidin-Tags, durch die exprimierte Fusionsproteine über Nickel-Chelat-Säuren gereinigt werden können, oder antigene Determinanten, die es erlauben, die Peptide über geeignete Antikörperaffinitätssäulen zu reinigen. Signalpeptide können für ein zuverlässiges Ausschleusen des erzeugten Peptides sorgen, während Reporterproteine, wie beispielsweise das eGFP (enhanced green fluorescent protein) eine optische Detektion des erzeugten Peptides ermöglicht.

Wie bereits erwähnt, kann die Herstellung des neuen Peptides durch chemische Synthese, auch Merrifield-Synthese genannt, oder mit molekularbiologischen Techniken erfolgen.

Vor diesem Hintergrund betrifft die Erfindung ferner ein Nukleinsäuremolekül mit einem für ein erfindungsgemäßes Peptid kodierenden Sequenzabschnitt, einen Expressionsvektor mit einem derartigen Nukleinsäuremolekül sowie gegebenenrfalls Kontrollsequenzen, insbesondere für Replikation, Transkription und/oder Translation, sowie eine Wirtszelle, die mit dem Expressionsvektor transfiziert oder transformiert ist.

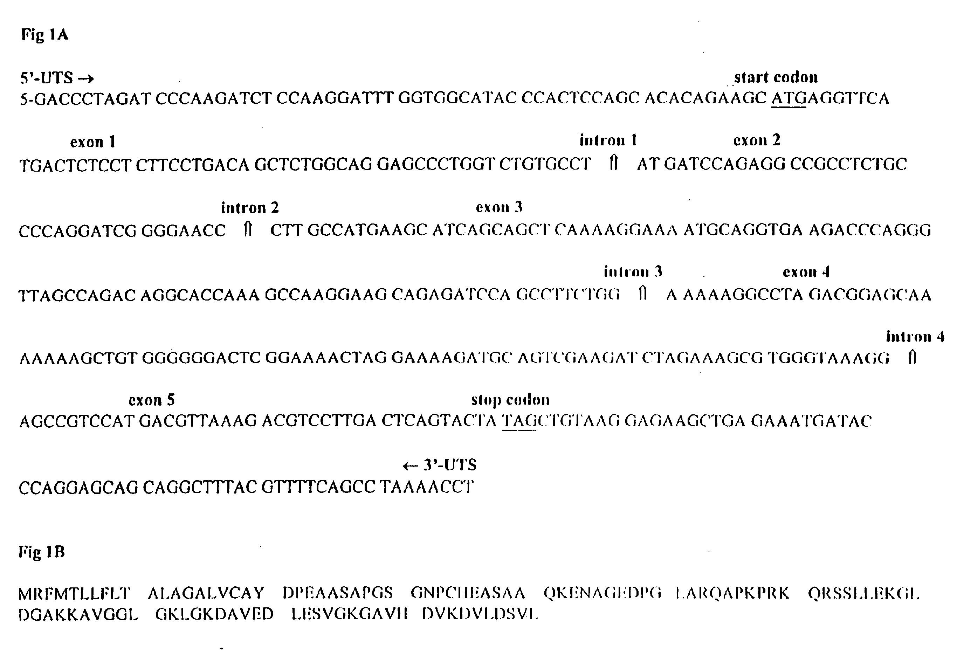

Nachdem die Aminosäuresequenz des erfindungsgemäßen Peptides bekannt ist, kann mit Hilfe des genetischen Codes eine entsprechende Nukleinsäuresequenz abgeleitet werden, wobei optimierte Codons für unterschiedliche Wirte (Bakterien, Hefe, Säugerzellen) verwendet werden können. Bevorzugt ist jedoch die sich aus Fig. 1 ergebende Codonwahl.

Die Herstellung eines erfindungsgemäßen Peptides durch Nukleinsäureexpression hat den Vorteil, daß das Peptid praktisch in unbegrenzten Mengen hergestellt werden kann. Darüber hinaus kann das Peptid aber auch auf einfache Art und Weise abgewandelt werden, indem nämlich die entsprechende kodierende Sequenz auf Nukleinsäureebene modifiziert wird, um so einen Aminosäureaustausch zu bewirken. Auf der Nukleinsäureebene können auch Sonden erzeugt werden, um in Schweißdrüsen anderer Säugerzellen nach homologen Peptiden zu suchen.

Wie bereits erwähnt, betrifft die Erfindung auch die Verwendung eines erfindungsgemäßen Peptides, das das DCD-Protein SEQ ID Nr: 1 oder ein vorzugsweise aus dem C-terminalen Bereich stammendes Fragment von DCD umfaßt, als antimikrobielles Agens.

Da DCD in Schweißdrüsen exprimiert wird, eignet es sich nach Erkenntnis der Erfinder besonders gut zum Schutz und zur Behandlung insbesondere humaner Haut.

Vor diesem Hintergrund betrifft die Erfindung ferner eine pharmazeutische bzw. kosmetische Zusammensetzung, die als wirksamen Bestandteil ein erfindungsgemäßes Peptid in einer antimikrobiell wirksamen Menge, vorzugsweise im Bereich von 1-50 µg/ml enthält.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Weitere Vorteile ergeben sich aus der nachfolgenden Beschreibung im Zusammenhang mit den Zeichnungen. Es zeigen:
- Fig. 1: in Fig. 1A die DNA-Sequenz für DCD und in Fig. 1B die Aminosäuresequenz;
- Fig. 2: in einem Balkendiagramm die mengenabhängige, anti-mikrobielle Wirkung des Fusionsproteins DCD-eGFP bei einer Inkubationszeit von 4 h gegenüber verschiedenen mikrobiellen Pathogenen;
- Fig. 3: in einem Balkendiagramm und einem experimentellen Ansatz wie Fig. 2 die antimikrobielle Wirkung des Peptides SEQ ID Nr: 2;
- Fig. 4: in einem Balkendiagramm die mengenabhängige und inkubationszeitabhängige antimikrobielle Wirkung des Peptides SEQ ID Nr: 2 gegenüber E. coli; und

- Fig. 5: in einem Balkendiagramm und einem Versuchsansatz wie in Fig. 3 die antimikrobielle Wirkung des Peptides SEQ ID Nr: 3.

### Beispiel 1: Isolation der DCD cDNA und Bestimmung der genomischen Sequenz

Beim Durchsuchen einer subtraktiven cDNA-Bibliothek von primärem Melanomgewebe und benignem Melanozyten-Naevusgewebe unter Verwendung von cDNA-Arrays wurde ein Klon isoliert, der verglichen mit dem Melanongewebe im Naevusgewebe überexprimiert war und der zum Zeitpunkt der Isolation keine Sequenzhomologie mit einem in der Genbank veröffentlichten Gen hatte; Hipfel et al., "Specifically Regulated Genes in Malignant Melanoma Tissues Identified by Subtractive Hybridization", British Journal of Cancer 82, 1149-1157 (2000). Der in der dortigen Veröffentlichung mit Klon 8 bezeichnete Klon wurde später Dermcidin (DCD) genannt.

Durch Sequenzierung von überlappenden PCR-Produkten wurde die volle Länge der DCD cDNA bestimmt, sie beträgt 458 bp mit einem offenen Leserahmen von 330 bp, der für 110 Aminosäurereste kodiert. Das Gen besteht aus fünf Exons und vier Introns und wird als Einzeltranskript exprimiert.

In Fig. 1A ist die genomische Sequenz des DCD-Gens für die fünf Exons angegeben, Fig. 1B zeigt die Peptidsequenz, die als SEQ ID Nr: 1 im Sequenzprotokoll zu finden ist. Von den 110 Aminosäureresten stellen die ersten 19 N-terminalen Aminosäurereste ein Signalpeptid dar.

DCD wurde dem Chromosom 12q13 zwischen den Markern D12S1896 und D12S1632 (lod score 14.11) zugeordnet. Das Molekulargewicht des unmodifizierten Proteins beträgt einschließlich Signalpeptid 11,2 kDa und ohne Signalpeptid 9,5 kDa.

### Beispiel 2: Nachweis von DCD in verschiedenen Gewebeproben

Um das Expressionsprofil von DCD zu bestimmen, wurde mit der Dot-Blot-Technik RNA von fünfzig verschiedenen Geweben und Entwicklungsstadien getestet, wobei markierte DCD cDNA als Sonde eingesetzt wurde. In keiner der fünfzig Proben wurde ein detektierbares Signal gefunden.

Um zu analysieren, ob das DCD-Gen nur in sehr geringem Maße in humanem Gewebe oder humanen Zellinien exprimiert wird, wurde eine RT-PCR für das DCD-Gen durchgeführt (Clontech MTC Panels). Es ergab sich, daß DCD stark in humaner Haut, humanem melanozytischem Naevusgewebe und Melanomgewebe exprimiert wird, daß jedoch in den sechzehn anderen analysierten humanen Geweben sowie in fötalem und in verschiedenen Tumorgeweben DCD nicht exprimiert wird. Darüber hinaus wurden auch nach einer RT-PCR mit vierzig Zyklen in verschiedenen Teilen des humanen Verdauungssystems und in verschiedenen Tumorzellinien keine Amplifikationsprodukte gefunden.

Aus diesen Ergebnissen läßt sich ableiten, daß die DCD-Expression auf Zellen in der Haut beschränkt ist.

Mittels *in situ* Hybridisierung, Immunhistochemie, Immunfluoreszenz und Immunelektronenmikroskopie wurde der Zelltyp bestimmt, der das DCD-Gen exprimiert.

Die *in situ* Hybridisierung ergab, daß das Gen in ekkrinen Schweißdrüsen innerhalb der Dermis der humanen Haut exprimiert wird. Bei Verwendung einer sense-Sonde für DCD als Negativkontrolle wurden keine Signale detektiert.

Für die Immunhistologie wurde ein DCD-Antiserum in Kaninchen erzeugt, wobei als antigene Determinante das Peptid KENAGEDPGL ARQAPKPRKQ RSSL verwendet wurde, das zur T-Zell-Stimulierung an KLH gekoppelt wurde. Die antigene Determinante entspricht der Aminosäuresequenz 42-65 aus dem DCD-Peptid. An den untersuchten Hautschnitten ergab sich eine starke Färbung der ekkrinen Schweißdrüsen, aber keine Expression auf anderen Zelltypen der Haut.

Für die Immunfluoreszenz wurden die Schnitte mit dem im vorstehenden Absatz angesprochenen polyklonalen Anti-DCD-Antiserum angefärbt und dann mit einem mit Cy5 (Dianova, Hamburg) markiertem Esel Anti-Kaninchen Antikörper inkubiert. Die myoepithelialen Zellen des sezernierenden Abschnittes der ekkrinen Schweißdrüsen wurde daraufhin mit einem mmonoklonalen Anti-Actin-Antikörper (Enzo Diagnostics, vertrieben von Loxo, Dossenheim, Deutschland) markiert, mit einem mit Cy3 markierten Esel Anti-Maus Antikörper (Dianova, Hamburg) gefärbt und alle Kerne mit YOPRO (Molecular Probes, Leiden, Niederlande) gefärbt. Die Schnitte wurden unter Verwendung eines konfokalen Laserabtastmikroskops (Leica TCS SP, Leica Microsystems, Benzheim) mit einer 250fachen Vergrößerung analysiert.

Es konnte eine starke Immunfluoreszenzanfärbung in den sezernierenden Abschnitten der ekkrinen Schweißdrüsen beobachtet werden. In sezernierenden Abschnitten von apokrinen Schweißdrüsen konnte nur ein schwaches und stark reduziertes Anfärben beobachtet werden.

Durch Immunelektronenmikroskopie konnte schließlich das DCD-Protein in den dunklen schleimsezernierenden Zellen des sezernierenden Abschnittes von ekkrinen Schweißdrüsen lokalisiert werden. Ultrastrukturell wurde DCD innerhalb des Golgi-Apparates und in den sezernierenden Körnern lokalisiert.

Bei einer Western-Blot-Analyse von humanem Schweiß wurden mit dem oben erwähnten Antiserum (Aminosäurereste 42-65 von DCD) drei Hauptproteinbanden bei ungefähr 17, 20 und 24 kDa detektiert. Das Protein mit dem höheren Molekulargewicht wurde nur in einigen Schweißproben detektiert, während die beiden anderen Banden auch dann detektiert werden, wenn Schweiß unter nichtreduzierenden Bedingungen analysiert wird.

Aus diesen Daten ergibt sich, daß in Schweißdrüsen DCD der vollen Länge in den dunklen schleimsezernierenden Zellen exprimiert und von dem Golgi-Apparat über sezernierende Körner zu der luminalen Fläche der Zellen transportiert wird, wo das Protein in den Kanal sezerniert wird. Aus der Western-Blot-Analyse konnte berechnet werden, daß die Menge an DCD-Protein in voller Länge im Schweiß zwischen 1 und 10 µg/ml liegt. Die in dem Schweiß detektierten drei Hauptproteinbanden, die mit dem DCD-Antiserum reagieren, scheinen unterschiedlich posttranslational modifizierte Formen des vollen DCD-Proteins zu sein.

### Beispiel 3: Konstruktion, Expression und Charakterisierung eines DCD-eGFP-Fusionsproteins

Die gesamte DCD cDNA ohne Stopcodon wurde in frame in den pEGFP-Vektor (Clontech, Heidelberg) 5' zu dem eGFP-Gen kloniert, wodurch ein DCD-eGFP genanntes Fusionsgen erzeugt wurde. Die korrekte Sequenz wurde durch Sequenzanalyse bestätigt.

SKMEL28 Melanomzellen (PNAS 73, 3278-3282 (1976)) wurden mit 1-2 µg DCD-eGFP oder eGFP allein unter Verwendung von Fugen (Roche, Mannheim) transfiziert und in RPMI mit 10 % FCS kultiviert.

Nach achtundvierzig Stunden wurden 500 µm/ml G418 (Calbiochem, Schwalbach) zu dem Medium hinzugegeben und die G418 Menge nach einer Woche auf 1 mg/ml geändert. Die Zellen wurden im Selektionsmedium gehalten und durch Grenzverdünnung kloniert. Für die weitere Analyse wurde ein stabiler Klon einer jeden Transfektion verwendet. Zellysate der beiden Klone (5-7 x10⁶ Zellen) wurden durch Inkubation der Zellen für dreißig Minuten in 1,2 ml Lysepuffer (PBS mit 0,5 % Triton X-100, 5 mM EDTA, 0,1 mM PMSF, 10 µM Pepstatin A, 10 µM Leupeptin, 25 µg/ml Aprotinin) präpariert. Die Lysate wurden von den Kernen nach Zentrifugation bei 12000 Upm für 20 min getrennt. Die von FCS, G418 und Penicillin/Streptomycin freien Überstände von transfizierten und nicht-transfizierten SKMEL28-Kontrollzellen wurden aufkonzentriert und durch Ultrafiltration mit einer Amicon-Filtrationszelle (10.000 Da) und Amicon Centricon Plus-20 Säulen mit einer Biomax-5 Membran (5.000 Da) entsalzt.

Im Western-Blot mit dem in Beispiel 2 beschriebenen Antiserum ergab sich im Zellysat ein Fusionsprotein von 44 kDa und in dem aufkonzentrierten Überstand zwei Proteine von 33 und 44 kDa. Das eGFP-Protein (24 kDa) wurde nur im Zellysat und nicht im Überstand gefunden.

Um die Aminosäurezusammensetzung von DCD weiter zu untersuchen, wurden die konzentrierten Überstände mit verschiedenen Proteasen (Sigma, Taufkirchen) inkubiert und die Proteine auf ein 11%iges SDS-Gel geladen. Mit einem Anti-GFP Antikörper wurden dann Western-Blots durchgeführt.

Die Proteasen Trypsin (300 µg/ml) und Chymotrypsin (300 µg/ml) wurden für 1 h bei 37 °C in 10 mM Tris-HCL, pH 8,0, enthaltend 2 mM CaCl₂ inkubiert. Zwei µl Endoproteinase ArgC (100 µg/ml) wurden in einem Puffer für 2 h bei 34°C inkubiert, der 0,1 M Tris-HCL pH 8.0, 8 mM CaCl₂, 50 mM DDT und 5 mM EDTA enthielt.

Der Proteaseverdau des Fusionsproteins zeigte, daß DCD-eGFP durch die Proteasen Trypsin und Chymotrypsin auf die Größe des GFP-Proteins abgedaut wird. Die Endoprotease ArgC, die an dem C-terminalen Ende von Argininresten schneidet, konnte das Fusionsprotein nicht abdauen, obwohl in dem reifen Protein (ohne Signalpeptid) voller Länge drei potentielle Schnittstellen vorhanden sind; siehe Fig. 1b, wo in den Positionen 53, 59 und 62 des reifen Proteins ein Arginin-Rest (R) zu erkennen ist.

Aus diesen Ergebnissen ergibt sich, daß die Schnittstelle für ArgC in dem Fusionsprotein nicht vorhanden ist. Da ferner das Fusionsprotein durch das DCD Antiserum aus Beispiel 2 im Western Blot nicht erkannt werden kann, ergibt sich, daß DCD proteolytisch prozessiert wird, um ein verkürztes Peptid zu ergeben, dem wenigstens die ersten 47 Aminosäurereste des sekretierten, reifen Proteins fehlt.

Mit anderen Worten, von dem DCD-Protein voller Länge wird im Schweiß ein Peptid erzeugt, das die letzten 48 C-terminalen Aminosäurereste (62-110) oder weniger enthält. Diesem Peptid fehlt ein Teil der für die für die Immunisierung verwendeten antigenen Determinante, es kann daher nicht durch das Antiserum aus Beispiel 2 detektiert werden. Obwohl ekkrine Schweißdrüsen mit dem Antiserum angefärbt werden können, war ein stabiles DCD-Protein mit dem antigenen Bereich nur in einigen Schweißproben zu finden. Auch das DCD-eGFP Fusionsprotein konnte mit dem Antiserum im aufkonzentrierten Überstand nicht nachgewiesen werden.

### Beispiel 4: Antimikrobielle Tests

Um die Eigenschaft von DCD bzw. der verkürzten DCD-Peptide weiter aufzuklären, wurde unter anderem die antimikrobielle Wirkung des Fusionsproteins DCD-eGFP, eines synthetisch hergestellten Peptides mit den 48 C-terminalen Aminosäureresten von DCD (SEQ ID Nr: 2) sowie eines aus einer HPLC-Fraktionierung von humanem Schweiß stammenden Peptides (SEQ ID Nr: 3) untersucht. Dieses letztere Peptid wies eine Masse von 4702 Dalton auf und entspricht bis auf das C-terminale Leucin der Sequenz SEQ ID Nr: 2. Dieses aus Schweiß isolierte Peptid ist im Sequenzprotokoll unter SEQ ID Nr: 3 aufgeführt.

Die antimikrobielle Wirkung der genannten Peptide wurde durch einen CFU-Test (Test auf koloniebildende Einheiten) durchgeführt, wie er für die Defensine beschrieben wurde von Valore et al., "Human Beta-Defensin-1: An Antimicrobial Peptide of Urogenital Tissues", J Clin Invest 101, 1633-1642 (1998).

Der Test der neuen Peptide wurde mit Escherichia coli, Staphylococcus aureus, Enterococcus faecalis und Candida albicans durchgeführt. E. coli wurde in LB-Medium, E. faecalis und S. aureus in Columbia-Medium (Difko, BD Heidelberg) und C. Albicans in Caseinhydrolysat-Medium (Merck, Darmstadt) inkubiert. Die Mengen der Bakterien und Hefen wurden photometrisch bestimmt. Die Bakterienstämme wurden bis zu einer optischen Dichte von 0,7 - 0,4 bei 600 nm und die Hefe bis zu einer optischen Dichte von 0,4 - 0,6 bei 450 nm inkubiert. Durch Plattieren verschiedener Verdünnungen wurde die Menge der Organismen wie folgt bestimmt:

Eine OD von 1 bei 600 nm entspricht 8,2 x 10⁹/ml für E. coli, 1,9 x 10¹⁰/ml für S. aureus und 9,0 x 10⁹/ml für E. faecalis, und ein OD von 1 bei 459 nm entspricht 1,4 x 10⁸/ml für C. albicans.

Die Zellen wurden zweimal mit 10 mM Natriumphosphatpuffer (pH 7,4) gewaschen und auf 2-3 x 10⁷ Zellen/ml (E. coli, E. faecalis), 5,7 x 10⁷ Zellen/ml (S. aureus) oder 5 x 10⁵ Zellen/ml (C. albicans) in Phosphatpuffer verdünnt.

Die Zellen wurden mit verschiedenen Mengen der Peptide in 200 µl Natriumphosphatpuffer bei 37 °C für 3 h, 4 h oder über Nacht (21 - 24 h) inkubiert. Die Zellen wurden für die Bakterien 1:10 bis 1:100 und für die Hefe 1:500 bis 1:5000 verdünnt und 50 µl, 100 µl und 200 µl auf entsprechende Agarplatten plattiert. Die Platten wurden über Nacht bei 37 °C inkubiert und die Anzahl der Kolonien gezählt. Die antimikrobielle Wirkung der Peptide wurde als Prozentsatz der getöteten Zellen angegeben: [1-(Zellüberleben nach Inkubation mit dem Peptid)/(Zellüberleben nach Kontrollinkubation)] x 100.

Zusätzlich zu dem Fusionsprotein sowie den Peptiden SEQ ID Nr: 2 und SEQ ID Nr: 3 wurde die antimikrobielle Wirkung von eGFP, Y-P30 (YDPEAASAPGSGNPCHEASAAQKENAGEDP, dies entspricht dem in der Beschreibung erwähnten Y-P30, in Position 23 jedoch mit dem Aminosäureaustausch K statt C, um eine hundertprozentige Homologie zu dem Aminosäureabschnitt 19-39 von DCD zu erhalten), sowie einem Kontrollpeptid DPI (DPYAEAASGPNPGSKSHESAQAENCGADPE) getestet.

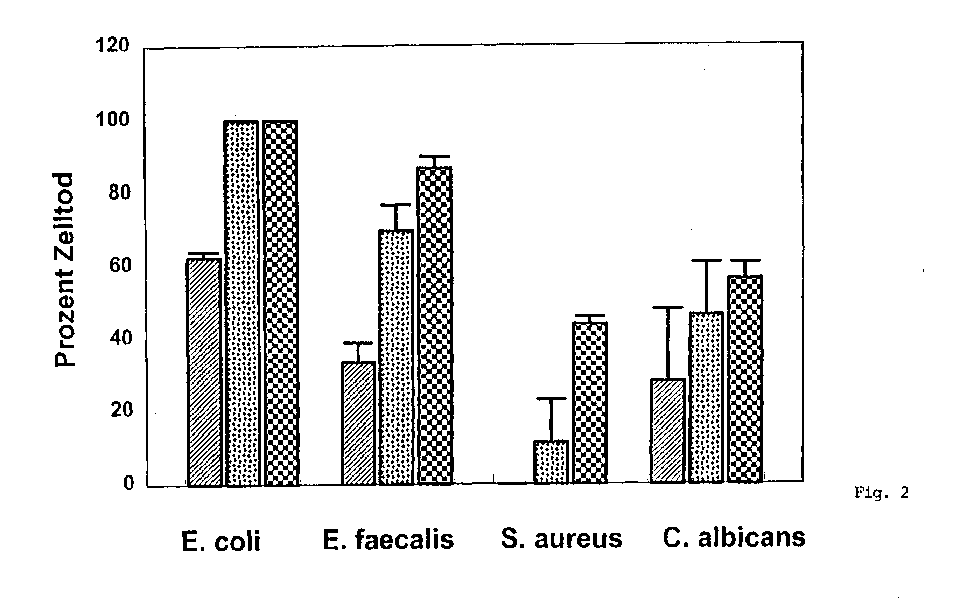

Während für eGFP, Y-P30 sowie DPI keine nennenswerte antimikrobielle Wirkung festgestellt werden konnte, zeigten das Fusionsprotein DCD-eGFP sowie die Peptide SEQ ID Nr: 2 und SEQ ID Nr: 3 eine mengenabhängige antimikrobielle Wirkung, wie sich aus den Fig. 2 bis 5 ergibt.

Fig. 2 zeigt die antimikrobielle Wirkung des Fusionsproteins DCD-eGFP (aus aufkonzentriertem Überstand) bei einer Inkubationszeit von 4 h gegenüber den vier genannten Pathogenen. Der linke Balken entspricht jeweils einer Peptidmenge von 0,1 µg/ml, der mittlere Balken einer Menge von 1 µg/ml und der rechte Balken einer Menge von 10 µg/ml.

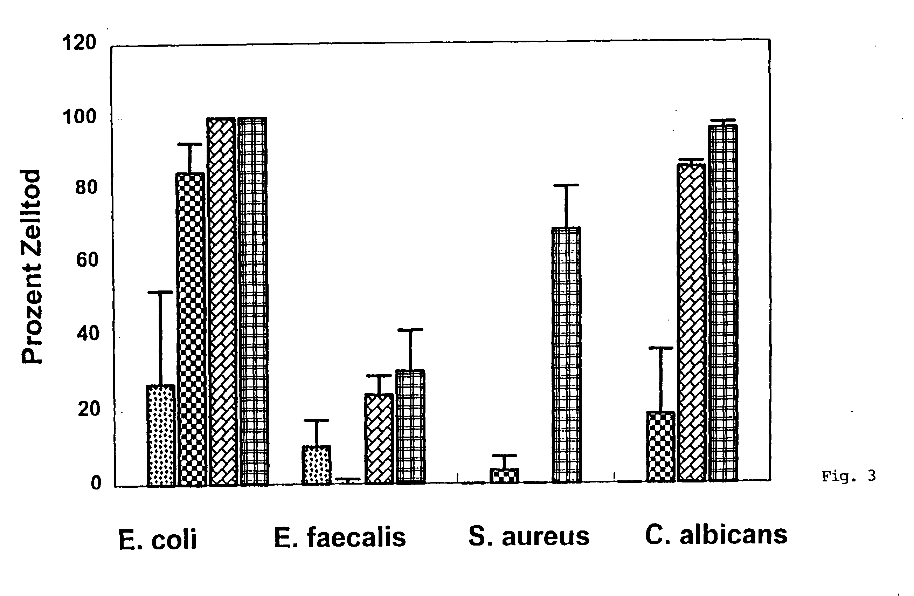

Fig. 3 zeigt die antimikrobielle Wirkung des Peptides SEQ ID Nr: 2 bei einer Inkubation von 4 h gegenüber den vier genannten Pathogenen, wobei die vier Balken von links nach rechts jeweils eine Menge von 1, 10, 50 bzw. 100 µg/ml entsprechen.

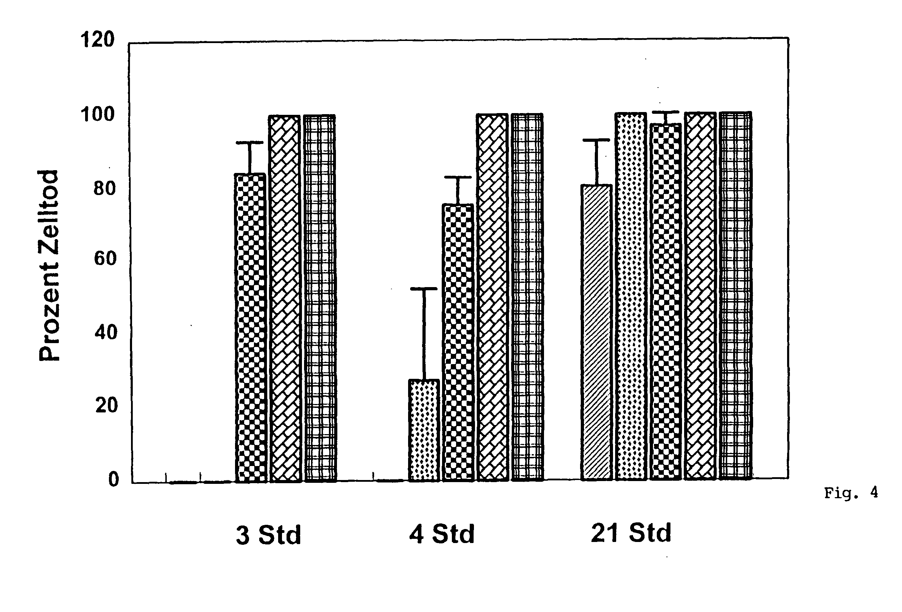

Fig. 4 zeigt die zeitabhängige Wirkung des Peptides SEQ ID Nr: 2 gegenüber E. coli, wobei die fünf Balken von links nach rechts jeweils eine Menge von 0,1, 1, 10, 50 bzw. 100 µg/ml entsprechen.

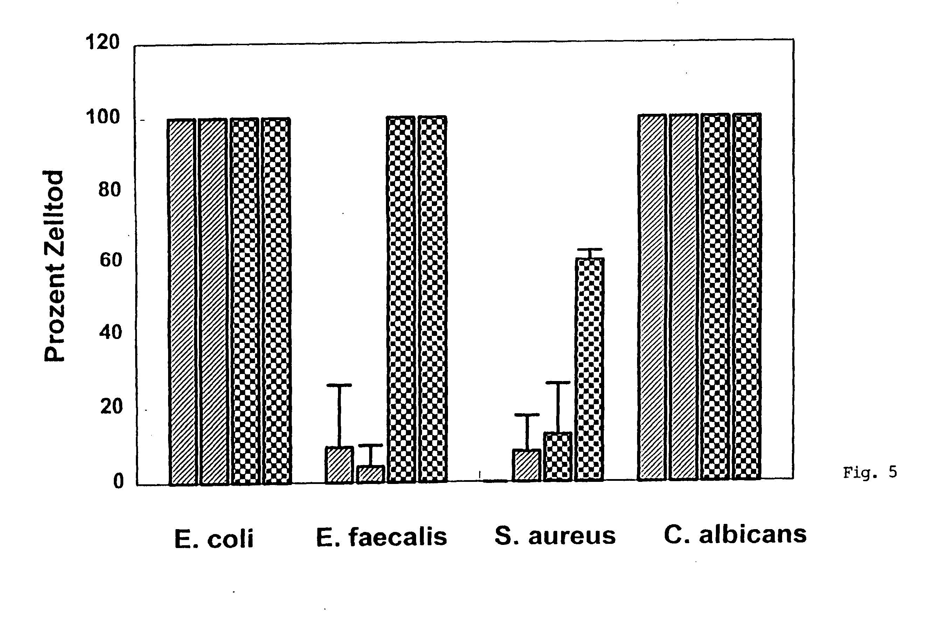

In Fig. 5 ist schließlich vergleichbar zu Fig. 2 die antimikrobielle Wirkung des Peptides SEQ ID Nr: 3 aus der HPLC-Fraktion bei einer Inkubation von 3 h bzw. 4 h gezeigt. Die beiden linken Balken entsprechen jeweils einer Menge von 2,5 µg/ml und die beiden rechten Balken einer Gruppe jeweils einer Menge von 25 µg/ml an eingesetztem Peptid. Der erste und dritte Balken einer Gruppe entspricht jeweils einer Inkubationszeit von 3 h, der zweite und vierte Balken einer Gruppe jeweils einer Inkubationszeit von 4 h.

Aus den Abbildungen 2 bis 5 ergibt sich, daß schon bei eingesetzten Peptidmengen, die der Menge an DCD im Schweiß entspricht (1-10 µg/ml), alle drei getesteten Peptide eine merkliche antimikrobielle Wirkung entfalten.

Aus dem Obigen ergibt sich, daß DCD sowie Fragmente von DCD eine antimikrobielle Wirkung gegenüber verschiedenen Pathogenen, beispielsweise Gram-positiven und Gram-negativen Bakterien sowie Hefen entfalten. Da DCD sowie die Fragmente von DCD in den Schweiß sezerniert werden und mit diesem auf die Hautoberfläche gelangen, sind die genannten Peptide zur kurativen oder protektiven Behandlung der Haut besonders geeignet, da sie dort offenbar ihre natürliche Wirkungsstätte haben.

## Patentansprüche

1. Antimikrobiell wirkendes Peptid, **dadurch gekennzeichnet, daß** es ein Fragment von höchstens 50 Aminosäureresten aus dem C-terminalen Bereich des DCD-Proteins SEQ ID Nr: 1 umfaßt.

2. Peptid nach Anspruch 1, **dadurch gekennzeichnet, daß** es die Aminosäurereste 63-110 von DCD umfaßt (SEQ ID Nr: 2).

3. Peptid nach Anspruch 1, **dadurch gekennzeichnet, daß** es die Aminosäurereste 63-109 von DCD umfaßt (SEQ ID Nr: 3).

4. Peptid nach einem der Ansprüche 1 bis 3, **dadurch gekennzeichnet, daß** verglichen mit der entsprechenden Position in DCD zumindest ein Aminosäurerest gegen einen Aminosäurerest der gleichen Gruppe ausgetauscht ist.

5. Antimikrobiell wirkendes Peptid, das eine zu dem Peptid nach einem der Ansprüche 1 bis 4 homologe Aminosäuresequenz aufweist und eine vergleichbare antimikrobielle Wirkung zeigt.

6. Peptid nach einem der Ansprüche 1 bis 5, **dadurch gekennzeichnet, daß** es zumindest eine posttranslationelle Modifizierung aufweist.

7. Peptid nach einem der Ansprüche 1 bis 6, **dadurch gekennzeichnet, daß** es mit einem weiteren Peptid oder Protein zu einem Fusionsprotein verbunden ist.

8. Peptid nach Anspruch 7, **dadurch gekennzeichnet, daß** das weitere Peptid oder Protein ausgewählt ist aus der Gruppe: Signalpeptid, Reporterprotein, Histidin-Tags, antigene Determinanten.

9. Nukleinsäuremolekül mit einem für ein Peptid nach einem der Ansprüche 1 bis 8 kodierenden Sequenzabschnitt.

10. Expressionsvektor mit dem Nukleinsäuremolekül nach Anspruch 9 und ggf. Kontrollsequenzen, insbesondere für Replikation, Transkription und/oder Translation.

11. Wirtszelle, die mit dem Expressionsvektor nach Anspruch 10 transfiziert oder transformiert ist.

12. Verwendung des Peptides nach einem der Ansprüche 1 bis 8 oder eines Peptides, das das DCD-Protein SEQ ID Nr: 1 oder ein vorzugsweise aus dem C-terminalen Bereich stammendes Fragment von DCD umfaßt, zur Herstellung eines Arzneimittels zur Behandlung von Krankheiten, die durch mikrobielle Pathogene hervorgerufen werden.

13. Verwendung des Peptides nach einem der Ansprüche 1 bis 8 oder eines Peptides, das das DCD-Protein SEQ ID Nr: 1 oder ein vorzugsweise aus dem C-terminalen Bereich stammendes Fragment von DCD umfaßt, zur Herstellung eines Arzneimittels zum Schutz vor oder zur Behandlung von hautkrankheiten.

14. Pharmazeutische Zusammensetzung, die als wirksamen Bestandteil ein Peptid nach einem der Ansprüche 1 bis 8 in - einer antimikrobiell wirksamen Menge enthält.

15. Kosmetische Zusammensetzung, die als wirksamen Bestandteil ein Peptid nach einem der Ansprüche 1 bis 8 in einer antimikrobiell wirksamen Menge enthält.

## Claims

1. An antimicrobially active peptide, **characterized in that** it comprises a fragment of a maximum of 50 amino acid residues from the C-terminal region of the DCD-protein SEQ ID No. 1.

2. The peptide as claimed in claim 1, **characterized in that** it comprises the amino acid residues 63-110 of DCD (SEQ ID No: 2).

3. The peptide as claimed in claim 1, **characterized in that** it comprises the amino acid residues 63-109 of DCD (SEQ ID No: 3).

4. The peptide as claimed in any of claims 1 to 3, **characterized in that**, compared with the corresponding position in DCD, at least one amino acid residue is exchanged for an amino acid residue of the same group.

5. An antimicrobially active peptide which comprises an amino acid sequence homologous to the peptide as claimed in any of claims 1 to 4 and shows a comparable antimicrobial effect.

6. The peptide as claimed in any of claims 1 to 5, **characterized in that** it has at least one post-translational modification.

7. The peptide as claimed in any of claims 1 to 6, **characterized in that** it is connected to a further peptide or protein to give a fusion protein.

8. The peptide as claimed in claim 7, **characterized in that** the further peptide or protein is selected from the group: signal peptide, reporter protein, histidine tags, antigenic determinants.

9. A nucleic acid molecule comprising a sequence segment encoding a peptide as claimed in any of claims 1 to 8.

10. An expression vector comprising the nucleic acid molecule as claimed in claim 9 and, where appropriate, control sequences, in particular for replication, transcription and/or translation.

11. A host cell which is transfected or transformed with the expression vector as claimed in claim 10.

12. Use of the peptide as claimed in any of claims 1 to 8 or of a peptide which comprises the DCD-protein SEQ ID No. 1 or a fragment of DCD, preferably derived from the C-terminal region of DCD, for the manufacture of a medicament for the treatment of diseases which are caused by microbial pathogens.

13. Use of the peptide as claimed in any of claims 1 to 8 or of a peptide which comprises the DCD-protein SEQ ID No. 1 or a fragment of DCD, preferably derived from the C-terminal region of DCD, for the manufacture of a medicament for the protection against or for the treatment of skin diseases.

14. A pharmaceutical composition which comprises as active ingredient a peptide as claimed in any of claims 1 to 8 in an antimicrobially effective amount.

15. A cosmetic composition which comprises as active ingredient a peptide as claimed in any of claims 1 to 8 in an antimicrobially effective amount.

## Revendications

1. Peptide à effet antimicrobien, **caractérisé en ce qu'**il comprend un fragment d'au plus 50 restes d'acide aminé de la zone C-terminale de la protéine DCD selon SEQ ID Nr : 1.

2. Peptide selon la revendication 1, **caractérisé en ce qu'**il comporte les restes d'acides aminés 63-110 de DCD (SEQ ID Nr : 2).

3. Peptide selon la revendication 1, **caractérisé en ce que** qu'il comporte les restes d'acides aminés 63-109 de DCD (SEQ ID Nr : 3).

4. Peptide selon l'une des revendications 1 à 3, **caractérisé en ce que**, comparé à la position correspondante dans le DCD, au moins un radical d'acide aminé est échangé contre un radical d'acide aminé du même groupe.

5. Peptide à effet antimicrobien qui présente une séquence d'acides aminés homologue au peptide selon l'une des revendications 1 à 4, et qui montre une activité antimicrobienne comparable.

6. Peptide selon l'une des revendications 1 à 5, **caractérisé en ce qu'**il présente au moins une modification post-traductionnelle.

7. Peptide selon l'une des revendications 1 à 6, **caractérisé en ce qu'**il est lié à un autre peptide ou à une protéine pour donner une protéine de fusion.

8. Peptide selon la revendication 7, **caractérisé en ce que** l'autre peptide ou protéine est choisi parmi le groupe : peptide de signal, protéine reporter, tags d'histidine, déterminants antigèniques.

9. Molécule d'acide nucléique avec un segment de séquence codant pour un peptide selon l'une des revendications 1 à 8.

10. Vecteur d'expression avec la molécule d'acide nucléique selon la revendication 9 et, le cas échéant, des séquences de contrôle, en particulier pour la réplication, la transcription et/ou la traduction.

11. Cellule hôte qui est transfectée ou transformée par le vecteur d'expression selon la revendication 10.

12. Utilisation d'un peptide d'après une des revendications 1 à 8 ou d'un peptide qui comprend la protéine DCD selon SEQ ID Nr : 1 ou un fragment de DCD provenant de préférence de la zone C-terminale, pour la préparation d'un médicament pour le traitement de maladies qui sont causées par des pathogènes microbiens.

13. Utilisation d'un peptide selon une des revendications 1 à 8 ou d'un peptide qui comprend la protéine DCD selon SEQ ID Nr : 1 ou un fragment de DCD provenant de préférence de la zone C-terminale pour la préparation d'un médicament pour la protection contre des maladies cutanées ou pour leur traitement.

14. Composition pharmaceutique qui contient comme composant actif un peptide selon une des revendications 1 à 8, en une quantité efficace du point de vue antimicrobien.

15. Composition cosmétique qui contient comme composant actif un peptide selon une des revendications 1 à 8 en une quantité efficace du point de vue antimicrobien.
